# FASCICULE DE BREVET EUROPEEN

(11) **EP 2 411 037 B1**
(45) Date de publication et mention de la délivrance du brevet: **12.04.2017**
(21) Numéro de dépôt: 10714961.9
(22) Date de dépôt: 29.03.2010
(51) Int. Cl.: A61K 38/28, A61K 47/61, A61P 5/50, A61K 47/36, A61K 9/00

(54) **FORMULATION D'INSULINE A ACTION RAPIDE**
SCHNELL WIRKENDE INSULINFORMULIERUNG
FAST-ACTING INSULIN FORMULATION

(30) Priorité: 27.03.2009 US 202692 P; 27.03.2009 FR 0901478
(43) Date de publication de la demande: 01.02.2012
(73) Titulaire: Adocia, 69003 Lyon (FR)
(72) Inventeur: SOULA, Gérard, F-69630 Meyzieu (FR); SOULA, Olivier, F-69630 Meyzieu (FR); SOULA, Rémi, 69330 Meyzieu (FR)
(74) Mandataire: Tripoz, Inès
(86) Numéro de dépôt international: PCT/IB2010/000711
(87) Numéro de publication internationale: WO 2010/122385

(56) Documents cités:
- WO-A-2007/116143
- WO-A-2008/038111
- FR-A- 2 224 164
- BAUDYS M ET AL: "Extending insulin action in vivo by conjugation to carboxymethyl dextran" BIOCONJUGATE CHEMISTRY, ACS, WASHINGTON, DC, US, vol. 9, no. 2, 5 février 1998 (1998-02-05) , pages 176-183, XP002363506 ISSN: 1043-1802

## Description

La présente invention concerne une formulation à action rapide d'insuline recombinante humaine.

Depuis la production d'insuline par génie génétique, au début des années 80, les patients diabétiques bénéficient d'insuline humaine pour se traiter. Ce produit a grandement amélioré cette thérapie puisque les risques immunologiques liés à l'utilisation d'insuline non humaine en particulier de porc se trouvent éliminés.

Un des problèmes à résoudre pour améliorer la santé des patients diabétiques est de mettre à leur disposition des formulations d'insuline qui permettent d'apporter une réponse hypoglycémiante s'approchant en terme de cinétique de la réponse physiologique générée par le début d'un repas pour leur permettre de ne pas anticiper leur heure de début de repas et de procéder à l'injection d'insuline au début du repas.

Aujourd'hui, il est admis que la mise à disposition de telles formulations est indispensable pour que la prise en charge de la maladie soit la meilleure possible.

Le génie génétique a permis d'apporter une réponse avec le développement d'insulines analogues rapides. Ces insulines sont modifiées sur un ou deux acides aminés pour être plus rapidement distribuées dans le compartiment sanguin après une injection sous-cutanée. Ces insulines Lispro (Lilly), Novolog (Novo) et Apidra (Aventis) sont des solutions stables d'insuline avec une réponse hypoglycémiante s'approchant en terme de cinétique de la réponse physiologique générée par le début d'un repas. Dès lors, les patients traités avec ces insulines analogues rapides n'ont plus à anticiper leur heure de repas, mais peuvent procéder à l'injection d'insuline au début du repas.

Le principe des insulines analogues rapides est de former des hexamères à la concentration de 100 UI/mL pour assurer la stabilité de l'insuline dans le produit commercial tout en favorisant la dissociation très rapide de ces hexamères en monomères après injection afin d'obtenir une action rapide.

L'insuline humaine telle que formulée sous sa forme commerciale, ne permet pas d'obtenir une réponse hypoglycémiante proche en terme de cinétique de la réponse physiologique générée par le début d'un repas, car à la concentration d'usage (100 UI/mL), en présence de zinc et d'autres excipients tel que le phénol ou le crésol, elle s'assemble sous forme d'hexamère alors qu'elle est active sous forme de monomère et de dimère. L'insuline humaine est préparée sous forme d'hexamères pour être stable près de 2 ans à 4°C car sous forme de monomères, elle a une très forte propension à s'agréger puis à fibriller ce qui lui fait perdre son activité. De plus sous cette forme agrégée, elle présente un risque immunologique pour le patient.

Les dissociations des hexamères en dimères et des dimères en monomères retardent son action de près de 20 minutes comparativement à une insuline analogue rapide (Brange J., et al, Advanced Drug Delivery Review, 35,1999, 307-335).

L'inconvénient des insulines analogues rapides est la modification de la structure primaire de l'insuline humaine. Cette modification entraîne des variations d'interaction avec les récepteurs de l'insuline présents sur un nombre très important de lignées cellulaires car il est connu que le rôle de l'insuline dans l'organisme n'est pas limité à son activité hypoglycémiante. Bien que de nombreuses recherches aient été effectuées dans ce domaine, on ne sait pas déterminer à ce jour si ces insulines analogues ont toutes les propriétés physiologiques de l'insuline humaine.

De plus, la cinétique de passage des insulines analogues dans le sang, ainsi que leur cinétique de réduction de la glycémie, ne sont pas optimales et il existe un réel besoin d'une formulation ayant un temps d'action encore plus court afin de s'approcher des cinétiques des patients sains.

La société Biodel a proposé une solution à ce problème avec une formulation d'insuline humaine comprenant de l'EDTA et de l'acide citrique telle que décrite dans la demande de brevet US200839365. L'EDTA par sa capacité à complexer les atomes de zinc et l'acide citrique par ses interactions avec les parties cationiques sont décrits comme déstabilisant la forme hexamérique de l'insuline et réduisant ainsi son temps d'action.

Cependant, une telle formulation présente plusieurs inconvénients.

Premièrement, l'injection d'une solution contenant de l'acide citrique peut provoquer des douleurs au site d'injection ce qui a été en effet, reporté au cours des différentes études cliniques réalisées par Biodel, Business Wire (Sep 08, 2008).

D'autre part, l'emploi d'un agent chélatant tel que l'EDTA, qui n'est pas spécifique de l'atome de zinc, peut entrainer des effets secondaires.

L'utilisation de l'insuline rapide s'effectuant trois fois par jour pour les diabétiques de type I et de type II, les douleurs associées à l'administration du produit ne sont pas acceptables pour les malades et les risques d'effets secondaires possibles dus aux excipients doivent être absolument évités.

Il existe donc un besoin réel et non satisfait de disposer de formulations permettant de réduire significativement le temps de début d'action (onset of action) de l'insuline injectée que celle-ci soit l'insuline humaine ou un analogue.

La présente invention permet de résoudre les différents problèmes ci-dessus exposés puisqu'elle permet notamment de réaliser une formulation d'insuline humaine à un pH compris entre 6,0 et 7,8, de préférence entre 6,5 et 7,5 en solution à 100 UI/mL, ladite formulation permettant, après administration, d'accélerer le passage de l'insuline dans le sang et/ou la réduction de la glycémie par rapport aux produits commerciaux d'insuline humaine.

Elle permet également de réduire significativement le temps de début d'action d'une formulation d'insuline analogue rapide.

L'invention telle que définie dans les revendications consiste à former un complexe d'insuline avec un polysaccharide comportant des groupes fonctionnels carboxyles partiellement substitués.

La formation de ce complexe peut de plus être réalisée par simple mélange d'une solution aqueuse d'insuline et d'une solution aqueuse de polysaccharide.

L'invention concerne également le complexe entre une insuline et un polysaccharide comportant des groupes fonctionnels carboxyles partiellement substitués.

Dans un mode de réalisation, l'insuline est l'insuline humaine.

On entend par insuline humaine une insuline obtenue par synthèse ou recombinaison dont la séquence peptidique est la séquence de l'insuline humaine, incluant les variations alléliques et les homologues.

Dans un mode de réalisation, l'invention concerne le complexe entre l'insuline humaine et un polysaccharide comportant des groupes fonctionnels carboxyles partiellement substitués.

Elle concerne également l'utilisation de ce complexe pour préparer des formulations d'insuline humaine permettant, après administration, d'accélerer le passage de l'insuline dans le sang et/ou la réduction de la glycémie par rapport aux produits commerciaux d'insuline humaine.

Les formulations d'insuline humaine « regular » sur le marché à une concentration de 600 µM (100 UI/mL) ont un délai d'action compris entre 30 et 60 minutes et un nadir glycémique compris entre 2 et 4 heures.

Les formulations d'insulines analogues rapides sur le marché à une concentration de 600 µM (100 UI/mL) ont un délai d'action compris entre 10 et 15 minutes et un nadir glycémique compris entre 60 et 90 minutes.

Elle concerne plus particulièrement l'utilisation d'un complexe selon l'invention pour la préparation d'une formulation d'insuline humaine dite rapide.

L'invention concerne l'utilisation du complexe selon l'invention pour préparer des formulations d'insuline humaine à une concentration voisine de 600 µM (100 UI/mL) dont le délai d'action est inférieur à 30 minutes, de préférence inférieur à 20 minutes, et encore de préférence inférieur à 15 minutes.

L'invention concerne l'utilisation du complexe selon l'invention pour préparer des formulations d'insuline humaine à une concentration voisine de 600 µM (100 UI/mL) dont le nadir glycémique est inférieur à 120 minutes, de préférence inférieur à 105 minutes, et encore de préférence inférieur à 90 minutes.

Dans un mode de réalisation, l'insuline est une insuline analogue. On entend par insuline analogue une insuline recombinante dont la séquence primaire contient au moins une modification par rapport à la séquence primaire de l'insuline humaine.

Dans un mode de réalisation l'insuline analogue est choisie dans le groupe constitué par l'insuline Lispro (Humalog^{®}), l'insuline Aspart (Novolog^{®}, Novorapid^{®}) et l'insuline glulisine (Apidra^{®}).

Dans un mode de réalisation, l'invention concerne le complexe entre une insuline analogue et un polysaccharide comportant des groupes fonctionnels carboxyles.

Dans un mode de réalisation, l'invention concerne le complexe entre une insuline analogue choisie dans le groupe constitué par l'insuline Lispro (Humalog^{®}), l'insuline Aspart (Novolog^{®}, Novorapid^{®)} et l'insuline glulisine (Apidra^{®}) et un polysaccharide comportant des groupes fonctionnels carboxyles.

Elle concerne également l'utilisation de ce complexe pour préparer des formulations d'insulines analogues permettant d'atteindre, après administration, un niveau plasmatique d'insuline et/ou une réduction du glucose plus rapidement que les formulations d'analogues de l'insuline.

Les formulations d'insulines analogues rapides sur le marché à une concentration de 600 µM (100 UI/mL) ont un délai d'action compris entre 10 et 15 minutes et un nadir glycémique compris entre 60 et 90 minutes.

Elle concerne plus particulièrement l'utilisation d'un complexe selon l'invention pour la préparation d'une formulation d'insuline analogue rapide.

L'invention concerne l'utilisation du complexe selon l'invention pour préparer des formulations d'insuline analogue à une concentration voisine de 600 µM (100 UI/mL) dont le délai d'action est inférieur à 15 minutes et de préférence inférieur à 10 minutes.

L'invention concerne l'utilisation du complexe selon l'invention pour préparer des formulations d'insuline analogue à une concentration voisine de 600 µM (100 UI/mL) dont le nadir glycémique est inférieur à 90 minutes et de préférence inférieur à 80 minutes.

Le polysaccharide constitué en majorité de liaisons glycosidiques de type (1,6) est un dextrane fonctionnalisé comportant des groupes fonctionnels carboxyles.

Lesdits polysaccharides sont fonctionnalisés par au moins un dérivé de la phénylalanine, noté Phe :
- ledit dérivé de la phénylalanine étant greffé ou lié aux polysaccharides par couplage avec une fonction acide, ladite fonction acide étant une fonction acide portée par un bras de liaison R lié au polysaccharide par une fonction F, ladite fonction F résultant du couplage entre le bras de liaison R et une fonction -OH du polysaccharide,
- F étant soit une fonction ester, thioester, carbonate, carbamate ou éther,
- R étant une chaîne comprenant entre 1 et 18 carbones, éventuellement branchée et/ou insaturée, comprenant un ou plusieurs hétéroatomes, tels que O, N ou/et S, et ayant au moins une fonction carboxyle,
- Phe étant un reste d'un dérivé de la phénylalanine, L ou D, produit du couplage entre l'amine de la phénylalanine et au moins un acide porté par le groupement R et/ou un acide porté par le polysaccharide comportant des groupes fonctionnels carboxyles.

Selon l'invention les polysaccharides fonctionnalisés répondent aux formules générales suivantes : 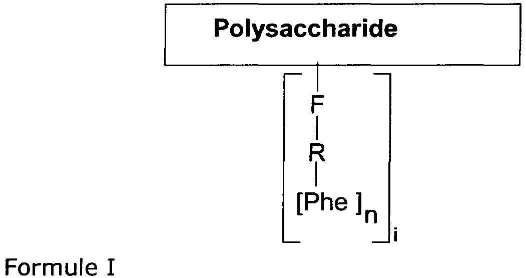
- le polysaccharide étant un dextrane,
- F résultant du couplage entre le bras de liaison R et une fonction - OH du polysaccharide et étant soit une fonction ester, thioester, carbonate, carbamate ou éther,
- R étant une chaîne comprenant entre 1 et 18 carbones, éventuellement branchée et/ou insaturée, comprenant un ou plusieurs hétéroatomes, tels que O, N ou/et S, et ayant au moins une fonction carboxyle,
- Phe étant un reste d'un dérivé de la phénylalanine, L ou D, produit du couplage entre l'amine du dérivé de la phénylalanine et au moins un acide porté par le groupement R et/ou un acide porté par le polysaccharide comportant des groupes fonctionnels carboxyles,
   n représente la fraction molaire des R substitués par Phe et est comprise entre 0,3 et 0,9, de préférence entre 0,4 et 0,8, encore de préférence entre 0,4 et 0,6,
   i représente la fraction molaire moyenne des groupements F-R-[Phe]ₙ portés par unité saccharidique et est comprise entre 0,5 et 2,5, de préférence entre 0,8 et 1,6, de préférence entre 1,0 et 1,4, de préférence entre 1,0 et 1,2 ;
      - lorsque R n'est pas substitué par Phe, alors le ou les acides du groupement R sont des carboxylates de cation, alcalin de préférence comme Na⁺ ou K⁺.

Dans un mode de réalisation, n qui représente la fraction molaire des R substitués par Phe est comprise entre 0,3 et 0,9, de préférence entre 0,4 et 0,8, encore de préférence entre 0,4 et 0,6,

Le polysaccharide comprend en moyenne au moins 60 motifs carboxylates substitués ou non substitués pour 100 motifs saccharidiques.

Dans un mode de réalisation, F est soit un ester, un carbonate, un carbamate ou un éther.

Dans un mode de réalisation, le polysaccharide selon l'invention est caractérisé en ce que le groupe R est choisi dans les groupes suivants : 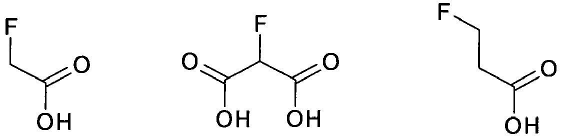 ou leurs sels de cations alcalins.

Dans un mode de réalisation, le polysaccharide selon l'invention est caractérisé en ce que le dérivé de la phénylalanine est choisi dans le groupe constitué par la phénylalanine et ses sels de cation alcalin, le phénylalaninol , le phénylalaninamide, l'éthyle benzyle amine.

Dans un mode de réalisation, le polysaccharide selon l'invention est caractérisé en ce que le dérivé de la phénylalanine est choisi parmi les esters de la phénylalanine de formule II

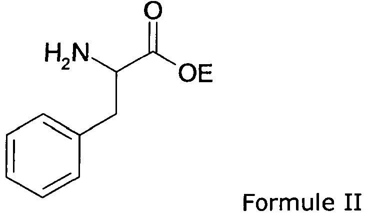

E étant un groupement alkyle linéaire ou ramifié en C1 à C6.

Le polysaccharide peut avoir un degré de polymérisation compris entre 10 et 3000.

Dans un mode de réalisation, il a un degré de polymérisation compris entre 10 et 400.

Dans un autre mode de réalisation, il a un degré de polymérisation compris entre 10 et 200.

Dans un autre mode de réalisation, il a un degré de polymérisation compris entre 30 et 50.

Dans un mode de réalisation, le polysaccharide à une masse comprise entre 9 et 50 kD, de préférence entre 10 et 40 kD.

Dans un mode de réalisation, l'insuline est une insuline humaine recombinante telle que décrite dans la Pharmacopée Européenne.

Dans un mode de réalisation, l'insuline est une insuline analogue choisie dans le groupe constitué par l'insuline Lispro (Humalog^{®}), l'insuline Aspart (Novolog^{®}, Novorapid^{®}) et l'insuline glulisine (Apidra^{®}).

Dans un mode de réalisation, les ratios molaires polymère/insuline sont compris entre 0,2 et 5.

Dans un mode de réalisation, ils sont compris entre 0,2 et 3.

Dans un mode de réalisation, ils sont compris entre 0,6 et 2,5.

mode de réalisationDans un mode de réalisation, ils sont compris entre 0,8 et 2.

Dans un mode de réalisation, ils ont compris entre 0,8 et 1,4.

Dans un mode de réalisation, le ratio molaire est égal à 1.

Dans un mode de réalisation, le ratio molaire est égal à 2.

Dans un mode de réalisation, les ratios massiques polymère/insuline sont compris entre 0,4 et 10.

Dans un mode de réalisation, ils sont compris entre 0,4 et 6.

mode de réalisationDans un mode de réalisation, ils sont compris entre 1,2 et 5.

Dans un mode de réalisation, ils sont compris entre 1,6 et 4.

mode de réalisationDans un mode de réalisation, ils sont compris entre 1,6 et 2,8.

De préférence cette composition est sous forme d'une solution injectable.

Dans un mode de réalisation, la concentration en insuline des solutions est de 600 µM soit 100 UI/mL.

Dans un mode de réalisation, la concentration en insuline de 600 µM peut être réduite par simple dilution, en particulier pour les applications pédiatriques.

L'invention concerne également une composition pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermiqueou intramusculaire.

Les voies d'administration transdermique, orale, nasale, vaginale, oculaire, buccale, pulmonaire sont également envisagées.

L'invention concerne également l'utilisation d'un complexe selon l'invention pour la formulation d'une solution d'insuline humaine de concentration de 100 UI/mL destinée aux pompes à insuline implantables ou transportables.

### Exemple 1 : Solution d'insuline analogue rapide à 100 UI/mL

Cette solution est une solution commerciale d'insuline Aspart commercialisée par la société Novo sous le nom de Novolog^{®} aux USA et Novorapid^{®}en Europe. Ce produit est une insuline analogue rapide.

### Exemple 2 ; Solution d'insuline humaine à 100 UI/mL

Cette solution est une solution commerciale de Novo vendue sous le nom d'Actrapid. Ce produit est une insuline humaine.

### Exemple 3 : Préparation d'une solution d'insuline humaine à 200 UI/mL

60,4 g d'eau sont ajoutés à 884,7 mg d'insuline humaine comprenant deux Zn²⁺ par hexamère, puis le pH est ajusté de 5,7 à 3 par ajout de 8 mL d'HCl 0,1 N. La solution est neutralisée à pH 7 par ajout de 10 mL de NaOH 0,1 N. La concentration est ensuite ajustée à 200 UI/mL par 43,08 mL d'eau. Le pH final de cette solution est de 7,02. La solution est enfin filtrée sur 0,22µm.

### Exemple 4 : Préparation des excipients

### Préparation du tampon phosphate 200 mM à pH 7

Une solution A de phosphate monosodique est préparée comme suit : 1,2 g de NaH₂PO₄ (10 mmol) sont solubilisés dans 50 mL d'eau dans une fiole jaugée.

Une solution B de phosphate disodique est préparée comme suit : 1,42 g de Na₂HPO₄ (10 mmol) sont solubilisés dans 50 mL d'eau dans une fiole jaugée.

Le tampon 200 mM phosphate à pH 7 est obtenu en mélangeant 3 mL de solution A à 7 mL de solution B.

### Préparation d'une solution de m-crésol 130 mM

La solution de m-crésol est obtenue en solubilisant 0,281 g de m-crésol (2,6 mmol) dans 20 mL d'eau dans une fiole jaugée.

### Préparation d'une solution de Tween 20 à 0,8 mM

La solution de Tween 20 est obtenue en solubilisant 98 mg de Tween 20 (80 µmol) dans 100 mL d'eau dans une fiole jaugée.

### Préparation d'une solution de Glycérine à 1,5 M

La solution de Glycérine est obtenue en solubilisant 13,82 g de glycérine (150 mmol) dans 100 mL d'eau dans une fiole jaugée.

### Préparation des solutions de polysaccharides

Deux polysaccharides selon l'invention sont employés.

Le polymère 1 est un dextraneméthylcarboxylate de sodium modifié par le sel de sodium de la L-phénylalanine obtenu à partir d'un dextrane de masse molaire moyenne en poids de 10 kg/mol, soit un degré de polymérisation de 39 (Pharmacosmos) selon le procédé décrit dans la demande de brevet FR07.02316. La fraction molaire moyenne de méthylcarboxylates de sodium, modifiés ou non par la phénylalanine, soit i dans la formule I, est de 1,06. La fraction molaire moyenne de méthylcarboxylates de sodium modifiés par la phénylalanine, soit n dans la formule I, est de 0,43.

La solution de polymère 1 est obtenue en solubilisant 2,79 g de polymère 1 (teneur en eau = 10%) dans 25,3 mL d'eau dans un tube de 50 mL (concentration en polymère 1 de 99,2 mg/mL).

Le polymère 2 est un dextraneméthylcarboxylate de sodium modifié par l'ester éthylique de la L-phénylalanine obtenu à partir d'un dextrane de masse molaire moyenne en poids de 40 kg/mol, soit un degré de polymérisation de 154 (Pharmacosmos) selon un procédé similaire à celui décrit pour le polymère 1 dans la demande de brevet FR07.02316 en employant le chlorhydrate de l'ester éthylique de la L-phénylalanine. La fraction molaire moyenne de méthylcarboxylates de sodium, modifiés ou non par la phénylalanine, soit i dans la formule I, est de 1,00. La fraction molaire moyenne de méthylcarboxylates de sodium modifiés par l'ester éthylique de la L-phénylalanine, soit n dans la formule I, est de 0,36.

La solution de polymère 2 est obtenue en solubilisant 1,33 g de polymère 2 (teneur en eau = 10%) dans 16,83 mL d'eau dans un tube de 50 mL (concentration en polymère 2 de 71,0 mg/mL).

Le polymère 3 est un dextraneméthylcarboxylate de sodium modifié par le sel de sodium de la L-phénylalanine obtenu à partir d'un dextrane de masse molaire moyenne en poids de 10 kg/mol, soit un degré de polymérisation de 39 (Pharmacosmos) selon le procédé décrit dans la demande de brevet FR07.02316. La fraction molaire moyenne de méthylcarboxylates de sodium, modifiés ou non par la phénylalanine, soit i dans la formule I, est de 1,06. La fraction molaire moyenne de méthylcarboxylates de sodium modifiés par la phénylalanine, soit n dans la formule I, est de 0,54.

La solution de polymère 3 est obtenue en solubilisant 1,5 g de polymère 3 (teneur en eau = 10%) dans 42,7 mL d'eau dans un tube de 50 mL (concentration en polymère 3 de 31,6 mg/mL).

Le polymère 4 est un dextraneméthylcarboxylate de sodium modifié par le sel de sodium de la L-phénylalanine obtenu à partir d'un dextrane de masse molaire moyenne en poids de 10 kg/mol, soit un degré de polymérisation de 39 (Pharmacosmos) selon le procédé décrit dans la demande de brevet FR07.02316. La fraction molaire moyenne de méthylcarboxylates de sodium, modifiés ou non par la phénylalanine, soit i dans la formule I, est de 1,69. La fraction molaire moyenne de méthylcarboxylates de sodium modifiés par la phénylalanine, soit n dans la formule I, est de 0,64.

La solution de polymère 4 est obtenue en solubilisant 2,0 g de polymère 4 (teneur en eau = 10%) dans 56,9 mL d'eau dans un tube de 50 mL (concentration en polymère 3 de 31,6 mg/mL).

### Exemple 5 : Préparation d'une solution d'insuline humaine à 100 UI/mL en présence de polymère 1

Pour un volume final de 50 mL de formulation avec un ratio molaire [polymère 1]/[insuline] de 1,0, les différents réactifs sont mélangés en quantités précisées dans le tableau ci-dessous et dans l'ordre qui suit :

| | |
|---|---|
| Insuline humaine à 200 UI/mL | 25 mL |
| Polymère 1 à 99,2 mg/mL | 3,61 mL |
| Tampon phosphate 1 M pH 7 | 500 µL |
| Tween 20 0,78 mM | 516 µL |
| Glycérine 1,5 M | 621 µL |
| m-crésol 130 mM | 11,15 mL |
| Eau (Volume pour dilution - volume de soude) | 8,55 mL |

Le pH final est de 7 ± 0,3.

Cette solution limpide est filtrée sur 0,22 µm puis est placée à +4°C.

### Exemple 6 : Préparation d'une solution d'insuline humaine à 100 UI/mL en présence de polymère 2

Pour un volume final de 50 mL de formulation avec un ratio molaire [polymère 2]/[insuline] de 0,5, les différents réactifs sont mélangés en quantités précisées dans le tableau ci-dessous et dans l'ordre qui suit :

| | |
|---|---|
| Insuline humaine à 200 UI/mL | 10 mL |
| Polymère 2 à 71,0 mg/mL | 9,66 mL |
| Tampon phosphate 1 M pH 7 | 500 µL |
| Tween 20 0,78 mM | 400 µL |
| Glycérine 1,5 M | 5,67 µL |
| m-crésol 130 mM | 11,16 mL |
| Eau (Volume pour dilution - volume de soude) | 12,6 mL |

Le pH final est de 7 ± 0,3.

Cette solution limpide est filtrée sur 0,22 µm puis est placée à +4°C.

### Exemple 7 : Préparation d'une solution d'insuline humaine à 100 UI/mL en présence de polymère 3

Pour un volume final de 50 mL de formulation avec un ratio molaire [polymère 3]/[insuline] de 1,0, les différents réactifs sont mélangés en quantités précisées dans le tableau ci-dessous et dans l'ordre qui suit :

| | |
|---|---|
| Insuline humaine à 500 UI/mL | 10 mL |
| Polymère 3 à 31,6 mg/mL | 11,9 mL |
| Tampon phosphate 1 M pH 7 | 500 µL |
| Glycérine 1,5 M | 5,67 mL |
| m-crésol 130 mM | 11,16 mL |
| Tween 20 1 mM | 0,4 mL |
| Eau (Volume pour dilution - volume de soude) | 10,4 mL |

Le pH final est de 7 ± 0,3.

Cette solution limpide est filtrée sur 0,22 µm puis est placée à +4°C.

### Exemple 8 : Préparation d'une solution d'insuline analogue à 100 UI/mL en présence de polymère 3

Pour un volume final de 10 mL de formulation avec un ratio molaire [polymère 3]/[insuline analogue] de 1,0, les différents réactifs sont mélangés en quantités précisées dans le tableau ci-dessous et dans l'ordre qui suit :

| | |
|---|---|
| Solution du produit commercial Novolog | 10 mL |
| Polymère 3 lyophilisé | 376 mg |
| Tween 20 | 98 µg |

Le pH final est de 7 ± 0,3.

Cette solution limpide est filtrée sur 0,22 µm puis est placée à +4°C.

### Exemple 9 : Préparation d'une solution d'insuline humaine à 100 UI/mL en présence de polymère 1

Pour un volume final de 50 mL de formulation avec un ratio molaire [polymère 1]/[insuline] de 2,0, les différents réactifs sont mélangés en quantités précisées dans le tableau ci-dessous et dans l'ordre qui suit :

| | |
|---|---|
| Insuline humaine à 200 UI/mL | 25 mL |
| Polymère 1 à 99,2 mg/mL | 7,22 mL |
| Tampon phosphate 1 M pH 7 | 500 µL |
| Tween 20 0,78 mM | 516 µL |
| Glycérine 1,5 M | 621 µL |
| m-crésol 130 mM | 11,15 mL |
| Eau (Volume pour dilution - volume de soude) | 4,94 mL |

Le pH final est de 7 ± 0,3.

Cette solution limpide est filtrée sur 0,22 µm puis est placée à +4°C.

### Exemple 10 : Préparation d'une solution d'insuline humaine à 100 UI/mL en présence de polymère 3

Une variante de la formulation d'insuline humaine avec le polymère 3 décrite à l'exemple 7 est réalisée en absence de phosphate. Cette solution a par ailleurs la même composition et un pH également de 7 ± 0,3.

### Exemple 11 : Préparation d'une solution d'insuline humaine à 100 UI/mL en présence de polymère 3

Une variante de la formulation d'insuline humaine avec le polymère 3 décrite à l'exemple 7 est réalisée en absence de phosphate et de Tween. Cette solution a par ailleurs la même composition et un pH également de 7 ± 0,3.

### Exemple 12 : Préparation d'une solution d'insuline humaine à 100 UI/mL en présence de polymère 4

Une variante de la formulation d'insuline humaine décrite à l'exemple 7 est réalisée en employant une solution du polymère 4 à la place de la solution du polymère 3. Cette solution a par ailleurs la même composition et un pH également de 7 ± 0,3.

### Exemple 13 : Injectabilité des solutions

Toutes ces solutions sont injectables avec les systèmes habituels d'injection d'insuline. Les solutions décrites dans les exemples 1, 2, 5 et 6 sont injectées toutes aussi facilement avec des seringues à insuline avec des aiguilles de 31 Gauge qu'avec des stylos à insuline de Novo, commercialisé sous le nom de Novopen, équipés d'aiguilles de 31 Gauge.

### Exemple 14 : Protocole de mesure de la Pharmacodynamie des solutions d'insuline

6 cochons domestiques d'environ 50 kg, préalablement cathétérisés au niveau de la jugulaire, sont mis à jeun 2 à 3 heures avant le début de l'expérience. Dans l'heure précédant l'injection d'insuline, 3 prélèvements sanguins sont réalisés afin de déterminer le niveau basal de glucose.

L'injection d'insuline à la dose de 0,125 UI/kg est réalisée en sous-cutané au niveau du cou, sous l'oreille de l'animal à l'aide du stylo à insuline Novopen équipé d'une aiguille 31 G.

Des prélèvements sanguins sont ensuite réalisés toutes les 10 minutes sur 3 heures puis toutes les 30 minutes jusqu'à 5 heures. Après chaque prélèvement, le cathéter est rincé avec une solution diluée d'héparine.

Une goutte de sang est prélevée pour déterminer la glycémie au moyen d'un glucomètre.

Les courbes de pharmacodynamie du glucose sont ensuite tracées.

### Exemple 15 : Résultats de Pharmacodynamie des solutions d'insuline

| **Exemple** | **Insuline** | **Polymère** | **ratio molaire Polymère/insuline** | **nombre de cochons** |
|---|---|---|---|---|
| 1 | Analogue | - | - | 24 |
| 2 | Humaine | - | - | 31 |
| 5 | Humaine | 1 | 1,0 | 24 |
| 6 | Humaine | 2 | 0,5 | 21 |
| 7 | Humaine | 3 | 1,0 | 9 |
| 8 | Analogue | 3 | 1,0 | 11 |
| 9 | Humaine | 1 | 2,0 | 5 |

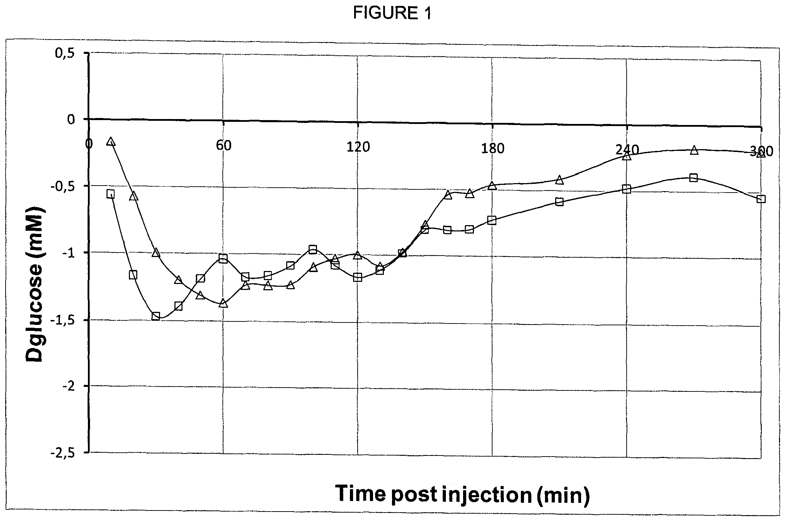

Les résultats obtenus avec la formulation d'insuline humaine décrite à l'exemple 5 sont représentés par les courbes de la figure 1. Les courbes montrent que la formulation de complexe entre le polymère 1 et l'insuline humaine selon l'invention (courbe avec les carrés correspondant à l'exemple 5) permet d'obtenir un délai d'action (onset of action) inférieur à celui d'une formulation commerciale d'insuline humaine (courbe avec les triangles correspondant à l'exemple 2).

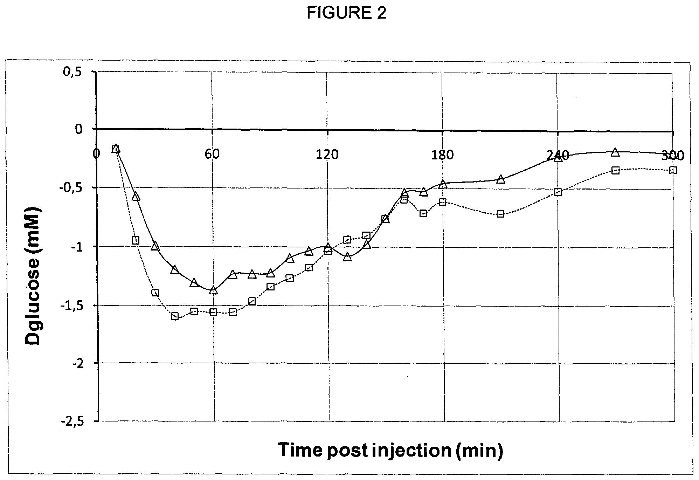

Les résultats obtenus avec la formulation d'insuline humaine décrite à l'exemple 6 sont représentés par les courbes de la figure 2. Les courbes montrent que la formulation du complexe entre le polymère 2 et l'insuline humaine selon l'invention (courbe avec les carrés correspondant à l'exemple 6) permettent d'obtenir un délai d'action (onset of action) inférieur à celui d'une formulation commerciale d'insuline humaine (courbe avec les triangles correspondant à l'exemple 2).

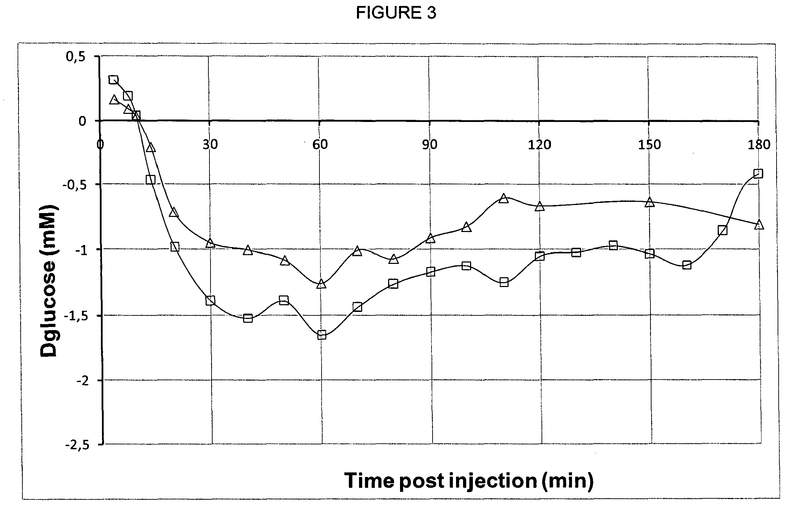

Les résultats obtenus avec la formulation d'insuline humaine décrite à l'exemple 7 sont représentés par les courbes de la figure 3. Les courbes montrent que la formulation du complexe entre le polymère 3 et l'insuline humaine selon l'invention (courbe avec les carrés correspondant à l'exemple 7) permettent d'obtenir un délai d'action (onset of action) inférieur à celui d'une formulation commerciale d'insuline humaine (courbe avec les triangles correspondant à l'exemple 2).

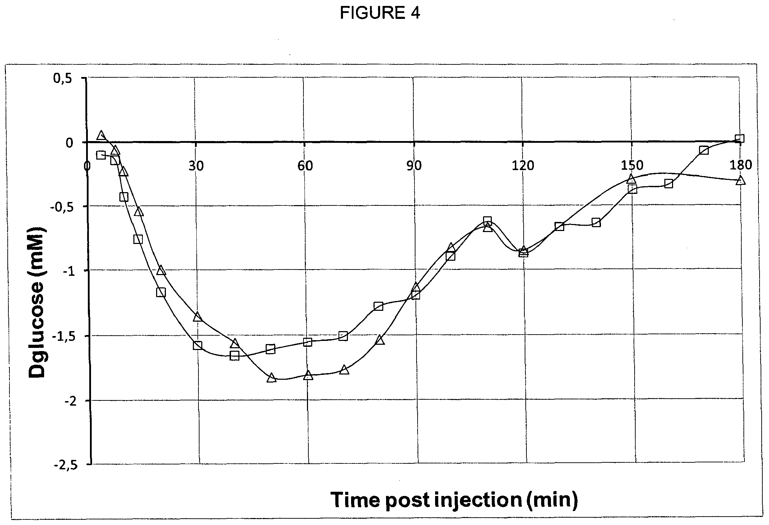

Les résultats obtenus avec la formulation d'insuline analogue décrite à l'exemple 8 sont représentés par les courbes de la figure 4. Les courbes montrent que la formulation du complexe entre le polymère 3 et une insuline analogue selon l'invention (courbe avec les carrés correspondant à l'exemple 8) permettent d'obtenir un délai d'action (onset of action) inférieur à celui d'une formulation commerciale de cette insuline analogue (courbe avec les triangles correspondant à l'exemple 1).

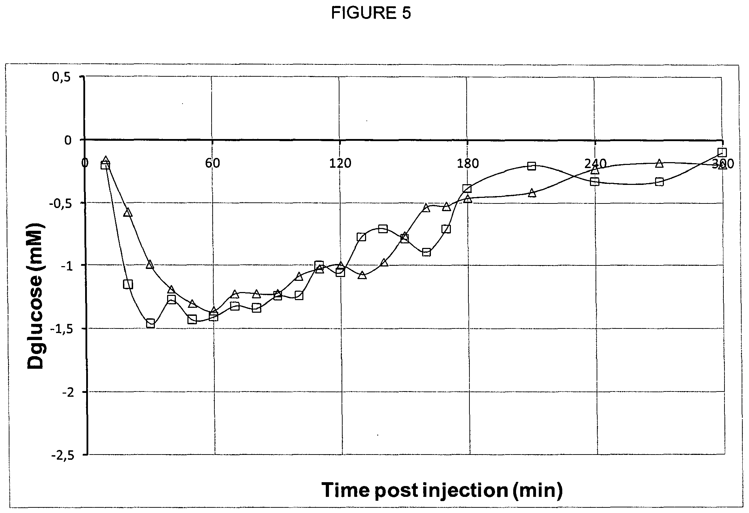

Les résultats obtenus avec la formulation d'insuline humaine décrite à l'exemple 9 sont représentés par les courbes de la figure 5. Les courbes montrent que la formulation du complexe entre le polymère 1 et l'insuline humaine selon l'invention (courbe avec les carrés correspondant à l'exemple 9) permettent d'obtenir un délai d'action (onset of action) inférieur à celui d'une formulation commerciale d'insuline humaine (courbe avec les triangles correspondant à l'exemple 2).

## Revendications

1. Complexe entre une insuline choisie parmi les insulines humaines recombinantes et les insulines analogues et un polysaccharide comportant des groupes fonctionnels carboxyles, ledit polysaccharide étant choisi parmi les polysaccharides fonctionnalisés de formule I : 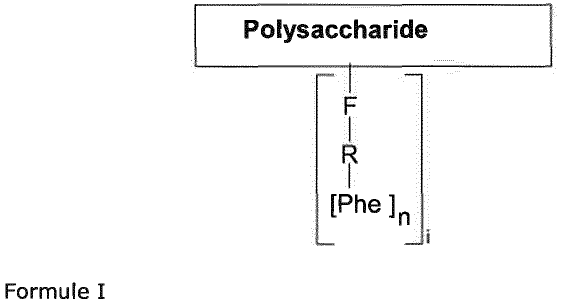
• le polysaccharide étant un dextrane,
• F résultant du couplage entre le bras de liaison R et une fonction -OH du polysaccharide et étant soit une fonction ester, thioester, carbonate, carbamate ou éther,
• R étant une chaîne comprenant entre 1 et 18 carbones, éventuellement branchée et/ou insaturée, comprenant un ou plusieurs hétéroatomes, tels que O, N ou/et S, et ayant au moins une fonction carboxyle,
• Phe étant un reste d'un dérivé de la phénylalanine, L ou D, choisi dans le groupe constitué par la phénylalanine et ses sels de cation alcalin, le phénylalaninol, le phénylalaninamide, l'éthyle benzyle amine, produit du couplage entre l'amine du dérivé de la phénylalanine et au moins un acide porté par le groupement R et/ou un acide porté par le polysaccharide comportant des groupes fonctionnels carboxyles,
• n représente la fraction molaire des R substitués par Phe et est comprise entre 0,3 et 0,9, de préférence entre 0,4 et 0,8, encore de préférence entre 0,4 et 0,6,
• i représente la fraction molaire moyenne des groupements F-R-[Phe]ₙ portés par unité saccharidique et est comprise entre 0,5 et 2,5, de préférence entre 0,8 et 1,6, de préférence entre 1,0 et 1,4, de préférence entre 1,0 et 1,2 ;
- lorsque R n'est pas substitué par Phe, alors le ou les acides du groupement R sont des carboxylates de cation, alcalin de préférence comme Na⁺ ou K⁺.

2. Complexe selon la revendication 1 **caractérisé en ce que** le groupe R est choisi dans les groupes suivants : 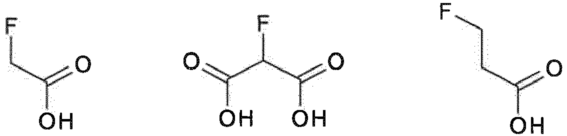 ou leurs sels de cations alcalins.

3. Complexe selon l'une quelconque des revendications précédentes **caractérisé en ce que** l'insuline est une insuline humaine recombinante.

4. Complexe selon l'une quelconque des revendications 1 à 2, **caractérisé en ce que** l'insuline est une insuline analogue.

5. Complexe selon la revendication 4, **caractérisé en ce que** l'insuline analogue est choisie dans le groupe constitué par l'insuline Lispro (Humalog^{®}), l'insuline Aspart (Novolog^{®}, Novorapid^{®}) et l'insuline glulisine (Apidra^{®}).

6. Complexe selon l'une quelconque des revendications 1 à 5 **caractérisé en ce que** les ratios massiques polymère/insuline sont compris entre 0,4 et 10.

7. Composition pharmaceutique comprenant au moins un complexe selon l'une quelconque des revendications 1 à 6.

8. Composition selon la revendication 7, **caractérisée en ce qu'**elle est sous forme d'une solution injectable.

9. Composition selon l'une quelconque des revendications 7 à 8 **caractérisée en ce que** la concentration en insuline des solutions est de 600 µmol/mL soit 100 UI/mL.

10. Utilisation d'un complexe selon l'une quelconque des revendications 1 à 3 et 6 pour la préparation d'une formulation d'insuline humaine à une concentration voisine en insuline de 600 µM (100 UI/mL) dont le délai d'action est inférieur à 30 minutes, de préférence inférieur à 20 minutes, et encore de préférence inférieur à 15 minutes.

11. Utilisation d'un complexe selon l'une quelconque des revendications 1 à 3 et 6 pour la préparation d'une formulation d'insuline humaine à une concentration en insuline voisine de 600 µmol/L (100 UI/mL) dont le nadir glycémique est inférieur à 120 minutes, de préférence inférieur à 105 minutes, et encore de préférence inférieur à 90 minutes.

12. Utilisation d'un complexe selon l'une quelconque des revendications 1 à 2 et 4 à 6 pour la préparation d'une formulation d'insuline analogue à une concentration voisine en insuline de 600 µM (100 UI/mL) dont le délai d'action est inférieur à 15 minutes, de préférence inférieur à 10 minutes.

13. Utilisation d'un complexe selon l'une quelconque des revendications 1 à 2 et 4 à 6 pour la préparation d'une formulation d'insuline analogue à une concentration en insuline voisine de 600 µmol/L (100 UI/mL) dont le nadir glycémique est inférieur à 90 minutes et de préférence inférieur à 80 minutes.

14. Utilisation d'un complexe selon l'une quelconque des revendications 1 à 6 pour la préparation d'une formulation d'insuline à 100 UI/mL destinée aux pompes à injection.

## Patentansprüche

1. Komplex zwischen einem Insulin, das ausgewählt ist aus rekombinanten menschlichen Insulinen und Insulinanalogen, und einem Polysacharid umfassend funktionelle Carboxylgruppen, wobei das Polysacharid ausgewählt ist aus den funktionalisierten Polysachariden von Formel I: 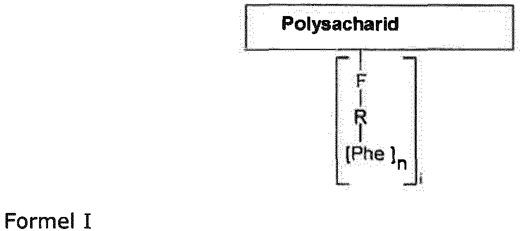 wobei
• das Polysacharid ein Dextran ist,
• F aus der Verknüpfung zwischen dem Linkerarm R und einer OH-Funktion des Polysacharids resultiert und entweder eine Ester-, Thioester-, Carbonat-, Carbamat oder Etherfunktion ist,
• R eine Kette ist umfassend zwischen 1 und 18 Kohlenstoffen, die optional verzweigt und/oder ungesättigt ist, umfassend ein oder mehrere Heteroatome wie beispielsweise O, N und/oder S, und mit mindestens einer Carboxylfunktion,
• Phe ein Rest eines Phenylalaninderivates, L oder D, ist, ausgewählt aus der Gruppe umfassend Phenylalanin und seine Alkalimetallkationsalze, Phenylalaninol, Phenylalaninamid, Ethylbenzylamin, hergestellt durch Verknüpfung zwischen dem Amin des Phenylalaninderivats und wenigstens einer Säure, die von der Gruppierung R getragen ist, und/oder einer Säure, die von dem Polysacharid getragen ist, das funktionelle Carboxylgruppen trägt,
• n der Molenbruch von mit Phe substituiertem R ist und zwischen 0,3 und 0,9, bevorzugt zwischen 0,4 und 0,8 und bevorzugter zwischen 0,4 und 0,6 beträgt,
• i der durchschnittliche Molenbruch der Gruppierungen F-R-[Phe]ₙ ist, die pro Saccharideinheit getragen sind, und zwischen 0,5 und 2,5, bevorzugt zwischen 0,8 und 1,6, bevorzugt zwischen 1,0 und 1,4, bevorzugt zwischen 1,0 und 1,2 beträgt;
wobei wenn R nicht mit Phe substituiert ist, dann ist die Säure oder sind die Säuren der Gruppierung R Carboxylate eines Kations, bevorzugt eines Alkalimetalls wie Na⁺ oder K⁺.

2. Komplex nach Anspruch 1, **dadurch gekennzeichnet, dass** die Gruppe R ausgewählt ist aus den folgenden Gruppen: 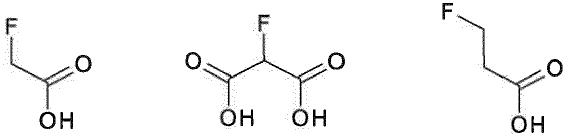 oder deren Alkalimetallkationensalze.

3. Komplex nach einem der vorhergehenden Ansprüche, **dadurch gekennzeichnet, dass** das Insulin ein rekombinantes humanes Insulin ist.

4. Komplex nach einem der Ansprüche 1 bis 2, **dadurch gekennzeichnet, dass** das Insulin ein Insulinanalog ist.

5. Komplex nach Anspruch 4, **dadurch gekennzeichnet, dass** das Insulinanalog ausgewählt ist aus der Gruppe umfassend das Insulin Lispro (Humalog^{®}), das Insulin Aspart (Novolog^{®}, Novorapid^{®}) und das Insulin Glulisin (Apidra^{®}).

6. Komplex nach einem der Ansprüche 1 bis 5, **dadurch gekennzeichnet, dass** die Massenverhältnisse Polymer / Insulin zwischen 0,4 und 10 betragen.

7. Pharmazeutische Zusammensetzung umfassend wenigstens einen Komplex nach einem der Ansprüche 1 bis 6.

8. Zusammensetzung nach Anspruch 7, **dadurch gekennzeichnet, dass** sie in Form einer injizierbaren Lösung vorliegt.

9. Zusammensetzung nach einem der Ansprüche 7 bis 8, **dadurch gekennzeichnet, dass** die Insulinkonzentration der Lösungen 600 µmol/ml, das heißt 100 IE/ml beträgt.

10. Verwendung eines Komplexes nach einem der Ansprüche 1 bis 3 und 6 für die Herstellung einer Formulierung von menschlichem Insulin mit einer Insulinkonzentration im Bereich von 600 µM (100 IE/ml), deren Wirkungseintritt weniger als 30 Minuten beträgt, bevorzugt weniger als 20 Minuten beträgt, und bevorzugter weniger als 15 Minuten beträgt.

11. Verwendung eines Komplexes nach einem der Ansprüche 1 bis 3 und 6 für die Herstellung einer Formulierung von menschlichem Insulin mit einer Insulinkonstellation im Bereich von 600 µM pro Liter (100 IE/ml), deren glyklämischer Tiefpunkt bei weniger als 120 Minuten, bevorzugt weniger als 105 Minuten, und bevorzugter weniger als 90 Minuten liegt.

12. Verwendung eines Komplexes nach einem der Ansprüche 1 bis 2 und 4 bis 6 für die Herstellung einer Formulierung eines Insulinanalogs mit einer Insulinkonzentration im Bereich von 600 µM (100 IE/ml), deren Wirkungseintritt weniger als 15 Minuten, bevorzugt weniger als 10 Minuten beträgt.

13. Verwendung eines Komplexes nach einem der Ansprüche 1 bis 2 und 4 bis 6 für die Herstellung einer Formulierung eines Insulinanalogs mit einer Insulinkonzentration im Bereich von 600 µmol/l (100 IE/ml), deren glykämischer Tiefpunkt bei weniger als 90 Minuten und bevorzugt weniger als 80 Minuten liegt.

14. Verwendung eines Komplexes nach einem der Ansprüche 1 bis 6 für die Herstellung einer Insulinformulierung mit 100 IE/ml für Injektionspumpen.

## Claims

1. A complex between an insulin chosen from the human, recombinant insulins and the analogue insulins and a polysaccharide comprising carboxyl functional groups, which polysaccharide is chosen from the functionalized polysaccharides of formula I : 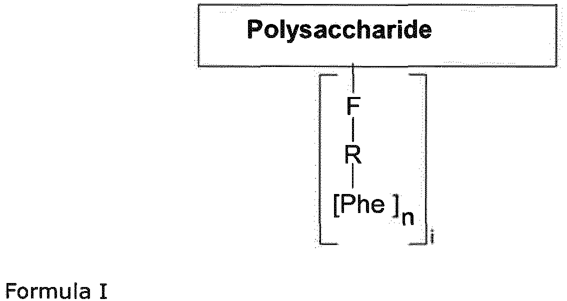
• The polysaccharide being a dextran,
• F resulting from the coupling between the arm of bond R and a -OH function of the polysaccharide and being either an ester, thioester, carbonate, carbamate or ether function,
• R being a chain comprising 1 to 18 carbons, optionally branched and/or unsaturated, comprising one or several heteroatoms such as O, N and/or S, and having at least one carboxyl function,
• Phe being a residue of a derivative of phenylalanine, L or D, selected from the group constituted by phenylalanine and its alkaline cation salts, phenylalaninol, phenylalaninamide, benzyl ethyl amine, produced from coupling between the amine of the derivative of the phenylalanine and at least one acid carried by the R group and/or an acid carried by the polysaccharide comprising functional carboxyl groups,
• n represents the molar fraction of the R's substituted by Phe and is comprised between 0.3 and 0.9, preferably between 0.4 and 0.8, or preferably between 0.4 and 0.6,
• i represents the average molar fraction of the groups F-R-[Phe]n carried by saccharidic unit and is comprised between 0.5 and 2.5, preferably between 0.8 and 1.6, preferably between 1.0 and 1.4, preferably between 1.0 and 1.2;
- when R is not substituted by Phe, then the acid or acids of the R group are alkaline cation carboxylates preferably like Na⁺ or K⁺.

2. The complex according to claim 1, **characterized in that** the R group is chosen from the following groups: 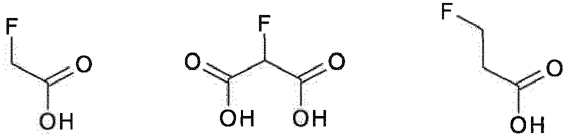 or their alkaline cation salts.

3. The complex according to any one of the previous claims, **characterized in that** the insulin is a human recombinant insulin.

4. The complex according to any one of Claims 1 to 2, **characterized in that** the insulin is an analogue insulin.

5. The complex according to Claim 4, **characterized in that** the analogue insulin is chosen from the group constituted by the Lispro insulin (Humalog^{®}), Aspart insulin (Novolog^{®}, Novorapid^{®}) and glulsine insulin (Apidra^{®}).

6. The complex according to any one of Claims 1 to 5, **characterized in that** the polymer/insulin mass ratios are comprised between 0.4 and 10.

7. A pharmaceutical composition comprising at least one complex according to any one of Claims 1 to 6.

8. The composition according to Claim 7, **characterized in that** it is in the form of an injectable solution.

9. The composition according to any one of Claims 7 to 8, **characterized in that** the concentration of insulin in the solutions is 600 µmol/ml or 100 UI/ml.

10. The use of a complex according to any one of Claims 1 to 3 and 6 for the preparation of a formulation of human insulin with a concentration of insulin close to 600 µM (100 UI/ml) whose onset of action is lower than 30 minutes, preferably lower than 20 minutes and even preferably lower than 15 minutes.

11. The use of a complex according to any one of Claims 1 to 3 and 6 for the preparation of a formulation of human insulin with a concentration of insulin close to 600 µmol/l (100 UI/ml) whose glycemic nadir is less than 120 minutes, preferably less than 105 minutes and even preferably than 90 minutes.

12. The use of a complex according to any one of Claims 1 to 2 and 4 to 6 for the preparation of a formulation of analogue insulin with a concentration of insulin close to 600 µM (100 UI/ml) whose onset of action is lower than 15 minutes, preferably lower than 10 minutes.

13. The use of a complex according to any one of Claims 1 to 2 and 4 to 6 for the preparation of a formulation of analogue insulin with a concentration of insulin close to 600 µmol/l (100 UI/ml) whose glycemic nadir is lower than 90 minutes and preferably lower than 80 minutes.

14. The use of a complex according to any one of Claims 1 to 6 for the preparation of a formulation of insulin at 100 UI/ml for injection pumps.
